# EUROPEAN PATENT SPECIFICATION

(11) **EP 4 180 073 B1**
(45) Date of publication and mention of the grant of the patent: **12.02.2025**
(21) Application number: 22204075.0
(22) Date of filing: 27.10.2022
(51) Int. Cl.: A61M 15/00, A61M 11/00

(54) **VERTICAL-TYPE MEDICINE CONTAINING NEBULIZING ASSEMBLY**
VERTIKAL-VERNEBELUNGSANORDNUNG FÜR MEDIKAMENTE
ENSEMBLE DE NÉBULISATION DE MÉDICAMENT DE TYPE VERTICAL

(30) Priority: 11.11.2021 CN 202111331508
(43) Date of publication of application: 17.05.2023
(73) Proprietor: Hcmed Innovations Co., Ltd., Taipei City 106 (TW)
(72) Inventor: TSAI, Wen-Yu, 106 Taipei City (TW); TSAI, Chien-Shen, 235 New Taipei City (TW)
(74) Representative: Viering, Jentschura & Partner mbB Patent- und Rechtsanwälte

(56) References cited:
- EP-A1- 1 219 314
- US-A1- 2006 243 274
- US-B1- 6 530 370
- US-B1- 6 769 626
- US-B2- 10 744 279

## Description

### CROSS-REFERENCE TO RELATED PATENT APPLICATION

This application claims the benefit of priority to China Patent Application No. 202111331508.6, filed on November 11, 2021 in People's Republic of China.

### FIELD OF THE DISCLOSURE

The present disclosure relates to a nebulizer and a medicine containing and nebulizing assembly thereof, and more particularly to a portable nebulizer and a vertical-type medicine containing and nebulizing assembly thereof.

### BACKGROUND OF THE DISCLOSURE

In the related art, a nebulizing method for the treatment of respiratory diseases is a more popular medical method in recent years. A nebulizer can be used in the nebulizing method to spray nebulized aerosols each has a particle size of about 3 to 5 µm, so that the nebulized aerosols can reach the bronchus and lungs for therapy. The procedure typically involves inhaling the nebulized aerosols from the medicinal liquid through mouths and noses of patients to enter the bronchus, and the particles of the nebulized aerosols are then spread to the whole alveolus so that the medicinal liquid can be sufficiently absorbed by the human body. This method of delivery is more direct and efficient than oral administration. Currently, the nebulizing method adapted by the nebulizer generally includes use of a pneumatic nebulization module, or an ultrasonic nebulization module (such as using a nebulizer mesh plate), configured for an operating mechanism of the nebulizer. However, there is still room for improvement in the related art for use of the nebulizer.

US 20060243274 A1 discloses a drug delivery device that uses an aerosol generator to nebulize a drug solution. The drug delivery device includes differently sized guide tubes and separator walls to provide substantially consistent particles that can be varied in size by using different guide tubes.

US 6530370 B1 discloses a nebulizer apparatus to atomize liquid solutions or suspensions. The nebulizer is typically used in conjunction with a breathing circuit to deliver atomized medicine to a patient.

US 6769626 B1 discloses an improved device and method for accurately controlling the supply of liquid in an apparatus discharging liquid. One such apparatus can comprise a nebulizer in which the accurate liquid supply enables the liquid to efficiently transformed into an aerosol.

EP 1219314 A1 discloses a liquid discharge apparatus, such as a nebulizer, discharges liquid droplets by supplying the liquid to a vibrating element having a plurality of openings.

US 10744279 B2 discloses an ultrasonic nebulizer includes: a working tank in which an ultrasonic vibrator is incorporated and in which a working liquid is stored facing the ultrasonic vibrator; a medicine tank that stores a medicinal liquid, at least a bottom portion thereof being dipped in the working liquid; and a main body that includes an oscillation circuit that is to drive the ultrasonic vibrator.

### SUMMARY OF THE DISCLOSURE

The invention is defined in the appended claims.

In response to the above-referenced technical inadequacy, the present disclosure provides a portable nebulizer and a vertical-type medicine containing and nebulizing assembly thereof.

In order to solve the above-mentioned problems, one of the technical aspects adopted by the present disclosure is to provide a vertical-type medicine containing and nebulizing assembly, which includes a medicine storage module, a nebulizing module and an induction module. The medicine storage module includes a medicine storage casing, and the medicine storage module has a medicine storage space formed in the medicine storage casing and a first through hole communicated with the medicine storage space. The nebulizing module includes a nebulizing device disposed in the medicine storage module and a first conductive structure electrically connected to the nebulizing device. The induction module includes at least one metal induction element disposed in the medicine storage module and a second conductive structure electrically connected to the at least one metal induction element. The medicine storage casing has an inner surrounding area located in the medicine storage space and surrounding the first through hole, and the at least one metal induction element has at least one exposed induction portion exposed on the inner surrounding area for receiving a signal generated by the nebulizing device.

In order to solve the above-mentioned problems, another one of the technical aspects adopted by the present disclosure is to provide a portable nebulizer, which includes a nebulizer main body, and a vertical-type medicine containing and nebulizing assembly disposed on the nebulizer main body and electrically connected to the nebulizer main body. The vertical-type medicine containing and nebulizing assembly includes a medicine storage module, a nebulizing module and an induction module. The medicine storage module includes a medicine storage casing, and the medicine storage module has a medicine storage space formed in the medicine storage casing and a first through hole communicated with the medicine storage space. The nebulizing module includes a nebulizing device disposed in the medicine storage module and a first conductive structure electrically connected between the nebulizing device and the nebulizer main body. The induction module includes at least one metal induction element disposed in the medicine storage module and a second conductive structure electrically connected between the at least one metal induction element and the nebulizer main body. The medicine storage casing has an inner surrounding area located in the medicine storage space and surrounding the first through hole, and the at least one metal induction element has at least one exposed induction portion exposed on the inner surrounding area for receiving a signal generated by the nebulizing device.

In one of the possible or preferred embodiments, the inner surrounding area has a first surrounding surface surrounding the first through hole, and a second surrounding surface surrounding the first surrounding surface. The inner surrounding area further has a plurality of liquid level measurement points and a plurality of liquid level induction portions are disposed adjacent to and along the liquid level measurement points. The liquid level measurement points are not located on the same horizontal plane and can be arranged on the first surrounding surface and/or the second surrounding surface, and the liquid level induction portions are not located on the same horizontal plane and can be arranged on the first surrounding surface and/or the second surrounding surface. The medicine storage casing has a first protruding blocking portion and a second protruding blocking portion that are disposed on the first surrounding surface, and the first protruding blocking portion is connected between the first through hole and the second protruding blocking portion. In addition, an upper surface of the exposed induction portion of the metal induction element is exposed by the first protruding blocking portion, and a side surface of the exposed induction portion of the metal induction element is covered by the first protruding blocking portion. Furthermore, an upper surface of the first protruding blocking portion is flush with or lower than the upper surface of the exposed induction portion, and an upper surface of the second protruding blocking portion is located above the upper surface of the exposed induction portion, in which the upper surface of the exposed induction portion of the metal induction element can be shown as circular, square, rectangular or arc shape.

In one of the possible or preferred embodiments, the inner surrounding area has a first surrounding surface surrounding the first through hole, and a second surrounding surface surrounding the first surrounding surface. The inner surrounding area further has a plurality of liquid level measurement points and a plurality of liquid level induction portions are disposed adjacent to and along the liquid level measurement points. The liquid level measurement points are not located on the same horizontal plane and can be arranged on the first surrounding surface and/or the second surrounding surface, and the liquid level induction portions are not located on the same horizontal plane and can be arranged on the first surrounding surface and/or the second surrounding surface. The medicine storage casing has a first protruding blocking portion and a second protruding blocking portion that are disposed on the first surrounding surface, and the first protruding blocking portion is connected between the first through hole and the second protruding blocking portion. In addition, the exposed induction portion of the metal induction element has an extending section disposed on the first protruding blocking portion, and a bending section extending downwardly from the extending section, and an upper surface of the extending section and an upper surface of the bending section are both exposed by the first protruding blocking portion, in which an upper surface of the first protruding blocking portion is flush with or lower than the upper surface of the extending section of the exposed induction portion, and an upper surface of the second protruding blocking portion is located above the upper surface of the extending section of the exposed induction portion.

In one of the possible or preferred embodiments, the inner surrounding area has a first surrounding surface surrounding the first through hole, and a second surrounding surface surrounding the first surrounding surface. The inner surrounding area further has a plurality of liquid level measurement points and a plurality of liquid level induction portions are disposed adjacent to and along the liquid level measurement points. The liquid level measurement points are not located on the same horizontal plane and can be arranged on the first surrounding surface and/or the second surrounding surface, and the liquid level induction portions are not located on the same horizontal plane and can be arranged on the first surrounding surface and/or the second surrounding surface. The medicine storage casing has a protruding blocking portion disposed on the second surrounding surface, and the protruding blocking portion is away from the first through hole by a predetermined distance, in which an upper surface and a top surrounding surface of the exposed induction portion of the metal induction element are exposed by the protruding blocking portion, and the upper surface of the exposed induction portion of the metal induction element can be shown as circular, square, rectangular or arc shape.

One beneficial effect of the present disclosure is that in the portable nebulizer and the vertical-type medicine containing and nebulizing assembly thereof provided by the present disclosure, when the predetermined medicinal liquid contained in the medicine storage space is nebulized through the nebulizing device, the signal generated by the nebulizing device can be transmitted to the microprocessor through the induction module, by virtue of "the medicine storage module including a medicine storage casing, and the medicine storage module having a medicine storage space formed in the medicine storage casing and a first through hole communicated with the medicine storage space," "the nebulizing module including a nebulizing device disposed in the medicine storage module and a first conductive structure electrically connected to the nebulizing device," "the induction module including at least one metal induction element disposed in the medicine storage module and a second conductive structure electrically connected to the at least one metal induction element," and "the medicine storage casing having an inner surrounding area located in the medicine storage space and surrounding the first through hole, and the at least one metal induction element having at least one exposed induction portion exposed on the inner surrounding area for receiving the signal generated by the nebulizing device."

These and other aspects of the present disclosure will become apparent from the following description of the embodiment taken in conjunction with the following drawings and their captions, although variations and modifications therein may be affected without departing from the scope of the novel concepts of the disclosure.

### BRIEF DESCRIPTION OF THE DRAWINGS

The described embodiments may be better understood by reference to the following description and the accompanying drawings, in which:
FIG. 1 is a schematic perspective exploded view of a vertical-type medicine containing and nebulizing assembly according to a first embodiment of the present disclosure;
FIG. 2 is a schematic perspective assembled view of the vertical-type medicine containing and nebulizing assembly according to the first embodiment of the present disclosure;
FIG. 3 is a schematic enlarged view of a portion III of FIG. 2;
FIG. 4 is a schematic cross-sectional view taken along line IV-IV of FIG. 2;
FIG. 5 is a schematic enlarged view of a portion V of FIG. 4;
FIG. 6 is a schematic perspective exploded view of a portable nebulizer according to the first embodiment of the present disclosure;
FIG. 7 is a schematic perspective assembled view of the portable nebulizer according to the first embodiment of the present disclosure;
FIG. 8 is a partial schematic cross-sectional view taken along line VIII-VIII of FIG. 7;
FIG. 9 is a schematic rear view of the portable nebulizer according to the first embodiment of the present disclosure;
FIG. 10 is a schematic view of an arc-shaped exposed induction portion of another metal induction element according to the first embodiment of the present disclosure;
FIG. 11 is a partial schematic perspective view of the vertical-type medicine containing and nebulizing assembly according to a second embodiment of the present disclosure;
FIG. 12 is a partial schematic cross-sectional view of the vertical-type medicine containing and nebulizing assembly according to the second embodiment of the present disclosure;
FIG. 13 is a schematic perspective exploded view of the vertical-type medicine containing and nebulizing assembly according to a third embodiment of the present disclosure;
FIG. 14 is a schematic perspective assembled view of the vertical-type medicine containing and nebulizing assembly according to the third embodiment of the present disclosure;
FIG. 15 is a schematic enlarged view of a portion XV of FIG. 14;
FIG. 16 is a schematic cross-sectional view taken along line XVI-XVI of FIG. 14;
FIG. 17 is a schematic enlarged view of a portion XVII of FIG. 16;
FIG. 18 is a schematic perspective assembled view of the vertical-type medicine containing and nebulizing assembly according to a fourth embodiment of the present disclosure;
FIG. 19 is a schematic enlarged view of a portion XIX of FIG. 18; and
FIG. 20 is a schematic perspective assembled view of the vertical-type medicine containing and nebulizing assembly according to a fifth embodiment of the present disclosure.

### DETAILED DESCRIPTION OF THE EXEMPLARY EMBODIMENTS

The present disclosure is more particularly described in the following examples that are intended as illustrative only since numerous modifications and variations therein will be apparent to those skilled in the art. Like numbers in the drawings indicate like components throughout the views. As used in the description herein and throughout the claims that follow, unless the context clearly dictates otherwise, the meaning of "a", "an" and "the" includes plural reference, and the meaning of "in" includes "in" and "on". Titles or subtitles can be used herein for the convenience of a reader, which shall have no influence on the scope of the present disclosure.

The terms used herein generally have their ordinary meanings in the art. In the case of conflict, the present document, including any definitions given herein, will prevail. The same thing can be expressed in more than one way. Alternative language and synonyms can be used for any term(s) discussed herein, and no special significance is to be placed upon whether a term is elaborated or discussed herein. A recital of one or more synonyms does not exclude the use of other synonyms. The use of examples anywhere in this specification including examples of any terms is illustrative only, and in no way limits the scope and meaning of the present disclosure or of any exemplified term. Likewise, the present disclosure is not limited to various embodiments given herein. Numbering terms such as "first", "second" or "third" can be used to describe various components, signals or the like, which are for distinguishing one component/signal from another one only, and are not intended to, nor should be construed to impose any substantive limitations on the components, signals or the like.

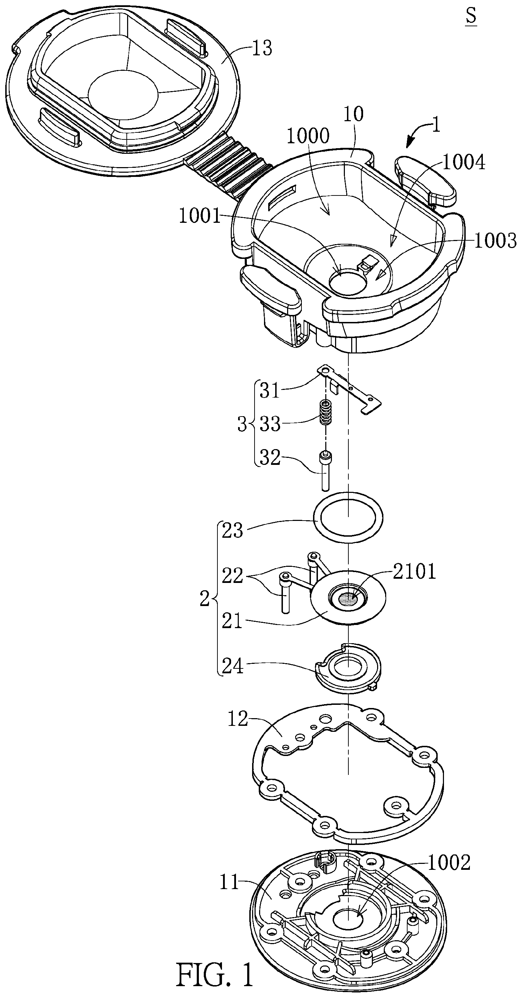

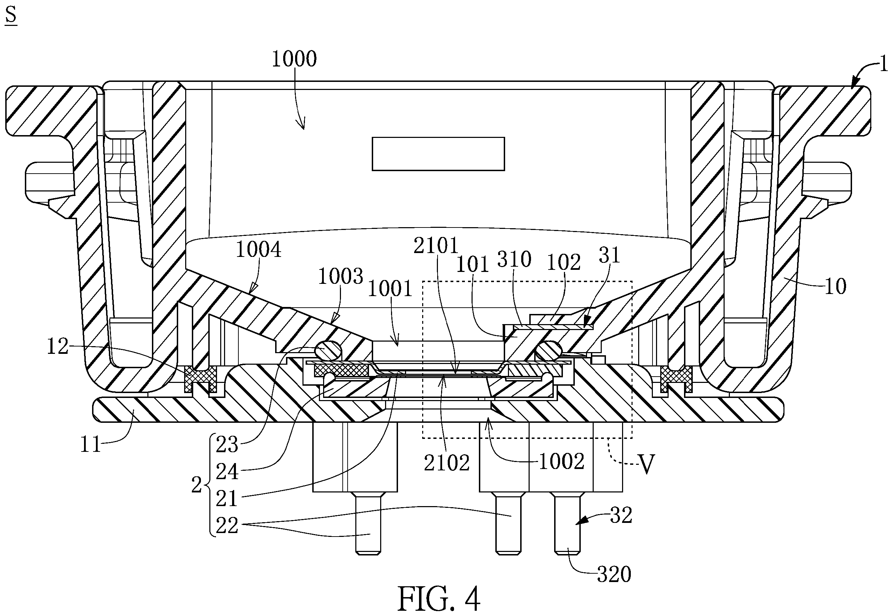

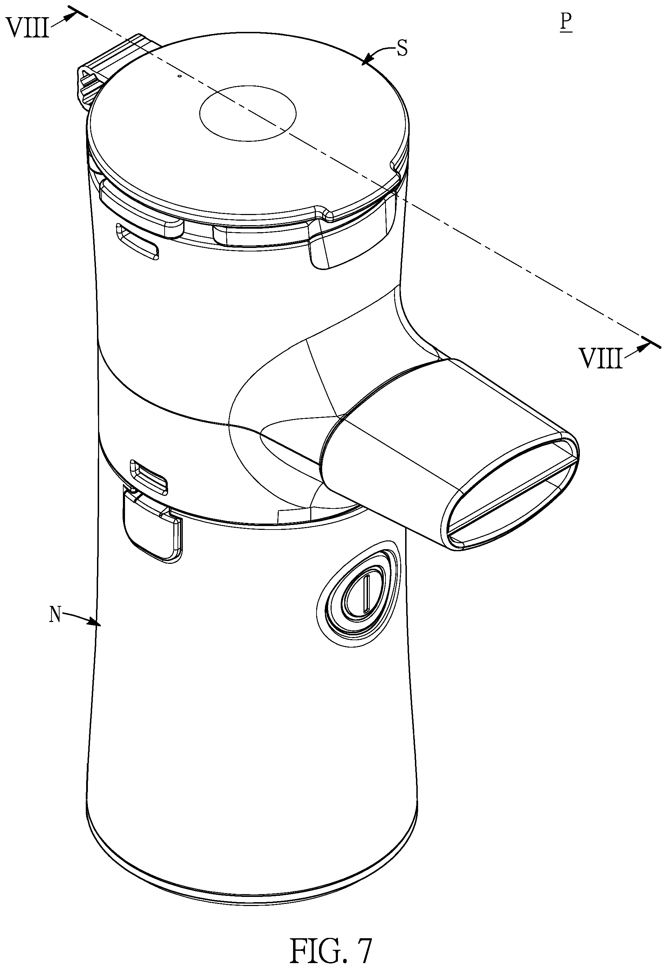

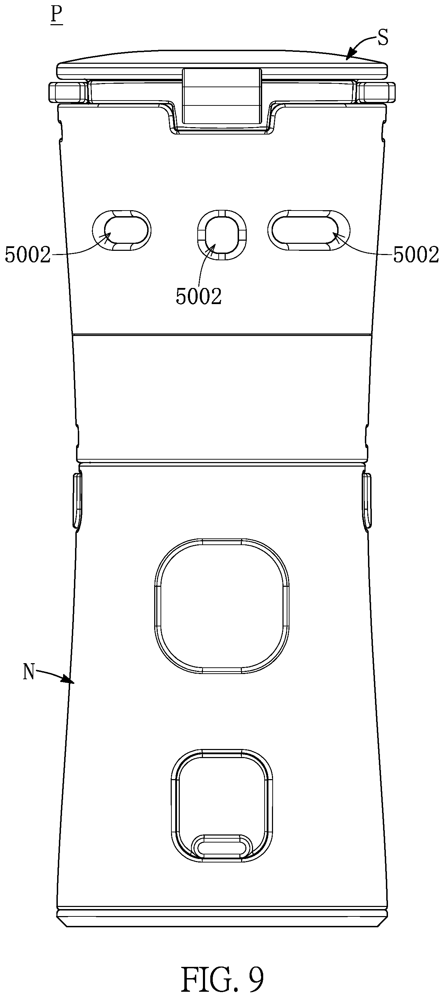

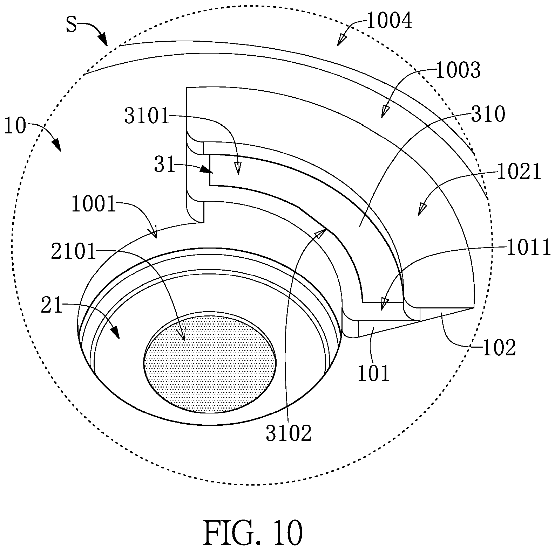

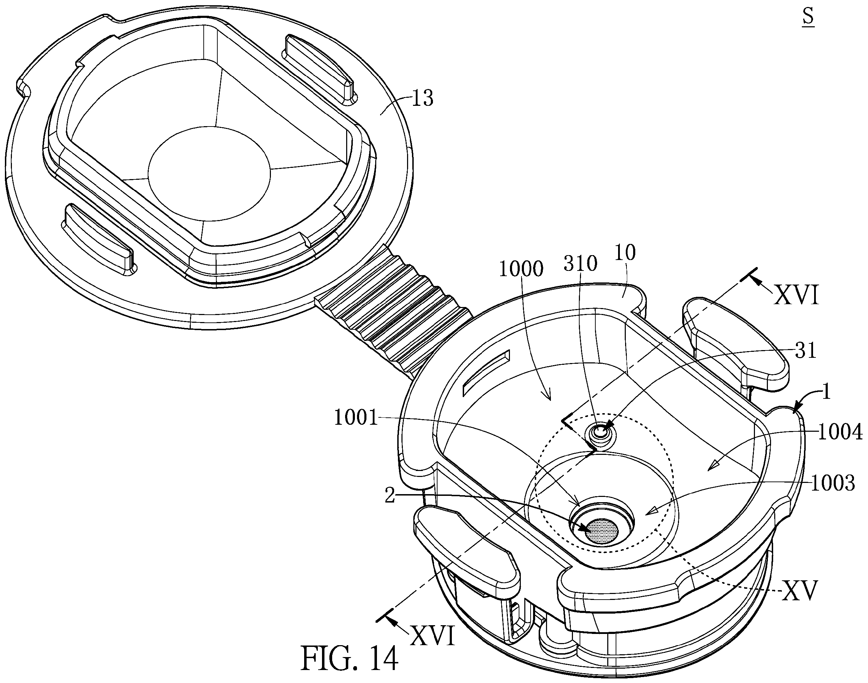

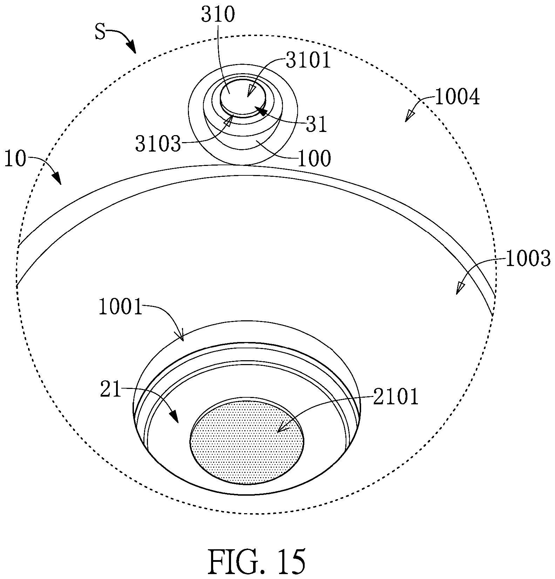

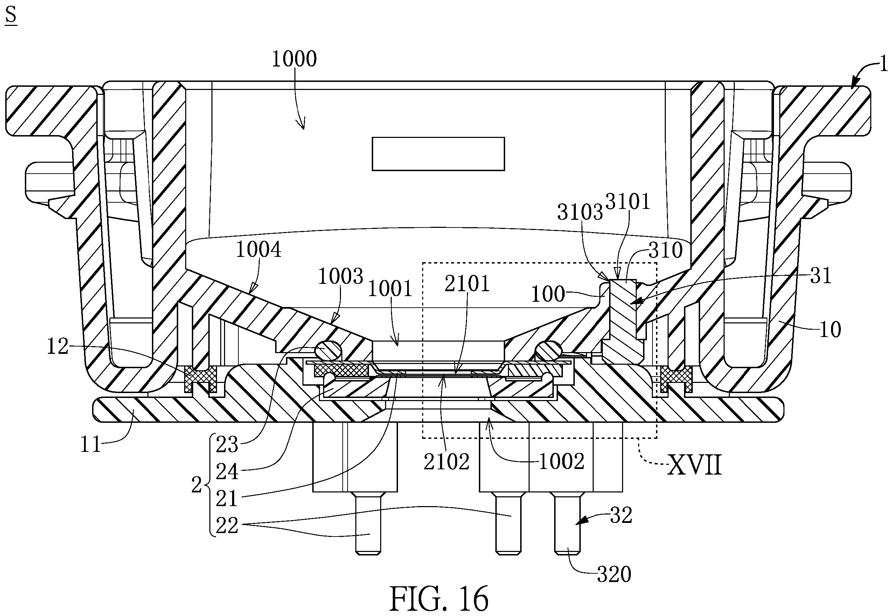

Referring to FIG. 1 to FIG. 17, the present disclosure provides a portable nebulizer P which includes a nebulizer main body N (such as a nebulizer main device) and a vertical-type medicine containing and nebulizing assembly S, and the vertical-type medicine containing and nebulizing assembly S is disposed on the nebulizer main body N and electrically connected to the nebulizer main body N. More particularly, the vertical-type medicine containing and nebulizing assembly S at least includes a medicine storage module 1 (such as a medicine containing module), a nebulizing module 2 and an induction module 3. The medicine storage module 1 includes a medicine storage casing 10 (such as a medicine containing casing), and the medicine storage module 1 has a medicine storage space 1000 (such as a medicine containing space) formed in the medicine storage casing 10 and a first through hole 1001 communicated with the medicine storage space 1000. The nebulizing module 2 includes a nebulizing device 21 disposed in the medicine storage module 1 and a first conductive structure 22 electrically connected between the nebulizing device 21 and the nebulizer main body N. The induction module 3 includes at least one metal induction element 31 disposed in the medicine storage module 1 and a second conductive structure 32 electrically connected between the at least one metal induction element 31 and the nebulizer main body N. It should be noted that the medicine storage casing 10 has an inner surrounding area located in the medicine storage space 1000 and surrounding the first through hole 1001, and the at least one metal induction element 31 has at least one exposed induction portion 310 exposed on the inner surrounding area for receiving a signal generated by the nebulizing device 21. In the present disclosure, the signal can be a contact signal (such as an electronic signal) or a non-contact signal (such as a radio frequency (RF) signal).

Therefore, when a predetermined medicinal liquid L (or a predetermined medicinal liquid) contained in the medicine storage space 1000 is nebulized through the nebulizing device 21, the signal generated by the nebulizing device 21 can be transmitted to a microprocessor 6 (such as an RF signal sensor) through the induction module 3. In a preferred embodiment, when the number of the exposed induction portions 310 exposed on the inner surrounding area is at least three, the three exposed induction portions 310 can be located on the same horizontal plane and surround the first through hole 1001, and the exposed induction portions 310 can be disposed on the same metal induction element 31 or different metal induction elements 31. Thereby, when the nebulizing device 21 is configured to be used for the nebulization, if the nebulizer main body N is placed upright, the predetermined medicinal liquid L contained in the medicine storage space 1000 can simultaneously contact the exposed induction portions 310, so that the signal generated by the nebulizing device 21 can be inducted by the exposed induction portions 310. In addition, when the nebulizing device 21 is configured to be used for the nebulization, if the nebulizer main body N is placed in an inclined position (for example, the nebulizer main body N is inclined relative to a vertical line at an inclination angle greater than or equal to 30 degrees), the predetermined medicinal liquid L contained in the medicine storage space 1000 will only contact a part of the exposed induction portion 310, so that the signal generated by the nebulizing device 21 will not be inducted by the exposed induction portion 310 that does not contact the predetermined medicinal liquid L, and the missing signal will not be transmitted to the microprocessor 6 by the induction module 3. Therefore, when the microprocessor 6 fails to receive the corresponding signal, the nebulizer main body N will remind the user to confirm the use state. However, the aforementioned details are disclosed for exemplary purposes only, and are not meant to limit the scope of the present disclosure.

Based on the above description, for example, when the volume of the predetermined medicinal liquid L in the medicine storage space 1000 is enough to contact the exposed induction portion 310, the signal generated by the nebulizing device 21 can be completely transmitted through the predetermined medicinal liquid L and received by the induction module 3, and then transmitted to the microprocessor 6 so as to use the microprocessor 6 to obtain a strength of a first signal. Furthermore, when the volume of the predetermined medicinal liquid L in the medicine storage space 1000 decreases and fails to contact the exposed induction portion 310, the signal generated by the nebulizing device 21 needs to be transmitted through the predetermined medicinal liquid L and the air in sequence and received by the induction module 3, or can only be transmitted through the air and received by the induction module 3, and then transmitted to the microprocessor 6 so as to use the microprocessor 6 to obtain a strength of a second signal, in which the strength of the second signal is less than the strength of the first signal. In a preferred embodiment, when the volume of the predetermined medicinal liquid L in the medicine storage space 1000 is decreased and does not contact the exposed induction portion 310, a residual volume of the predetermined medicinal liquid will be equal to or less than 0.4 ml. Therefore, when the difference between the strength of the first signal and the strength of the second signal is greater than a predetermined differential value, or when the strength of the second signal is equal to or lower than a strength of a predetermined signal, the nebulizer main body N is configured to stop supplying the power to the nebulizing device 21 so as to achieve the effect of automatically turning off the nebulizing device 21 (or automatically turning off the portable nebulizer P). However, the aforementioned details are disclosed for exemplary purposes only, and are not meant to limit the scope of the present disclosure.

### [First Embodiment]

Referring to FIG. 1 to FIG. 8, a first embodiment of the present disclosure provides a vertical-type medicine containing and nebulizing assembly S, and a portable nebulizer P using the vertical-type medicine containing and nebulizing assembly S, and the vertical-type medicine containing and nebulizing assembly S includes a medicine storage module 1, a nebulizing module 2 and an induction module 3.

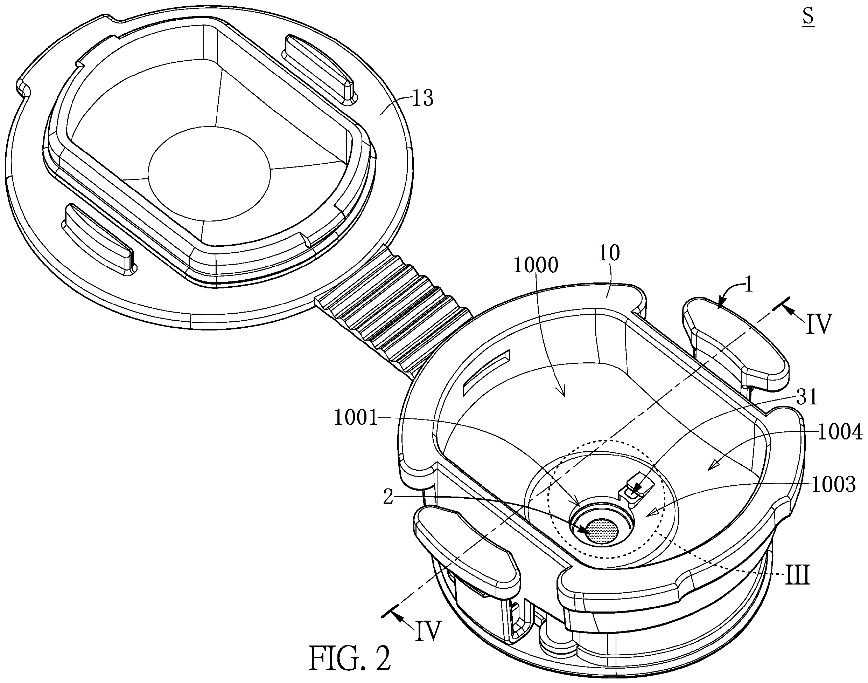

Firstly, referring to FIG. 1 and FIG. 2, the medicine storage module 1 includes a medicine storage casing 10, the medicine storage module 1 has a medicine storage space 1000 formed in the medicine storage casing 10 and a first through hole 1001 communicated with the medicine storage space 1000, and the medicine storage casing 10 has an inner surrounding area located in the medicine storage space 1000 and surrounding the first through hole 1001. For example, the medicine storage module 1 includes a carrier seat 11 configured to cooperate with the medicine storage casing 10, a waterproof element 12 (such as a waterproof ring) disposed between the medicine storage casing 10 and the carrier seat 11, and a movable cover 13 movably disposed on the medicine storage casing 10, the medicine storage module 1 has a second through hole 1002 passing through the carrier seat 11, and the medicine storage space 1000 is formed between the movable cover 13 and the medicine storage casing 10. However, the aforementioned details are disclosed for exemplary purposes only, and are not meant to limit the scope of the present disclosure.

Moreover, referring to FIG. 1, FIG. 2 and FIG. 4, the nebulizing module 2 includes a nebulizing device 21 disposed in the medicine storage module 1 and a first conductive structure 22 (or a first electrode structure) electrically connected to the nebulizing device 21. For example, the nebulizing device 21 can be used to convert the predetermined medicinal liquid contained in the medicine storage space 1000 into nebulized aerosols through a nebulizer mesh plate. In addition, the nebulizing module 2 includes a first waterproof washer 23 disposed between the medicine storage casing 10 and the nebulizing device 21, and a second waterproof washer 24 disposed between the carrier seat 11 and the nebulizing device 21, and the nebulizing device 21 has an inlet opening 2101 communicated with the first through hole 1001 and an outlet opening 2102 communicated with the second through hole 1002. However, the aforementioned details are disclosed for exemplary purposes only, and are not meant to limit the scope of the present disclosure.

Furthermore, referring to FIG. 1, FIG. 3 and FIG. 4, the induction module 3 includes at least one metal induction element 31 disposed in the medicine storage module 1 and a second conductive structure 32 (or an electrode structure) electrically connected to the at least one metal induction element 31, and the metal induction element 31 has at least one exposed induction portion 310 exposed on the inner surrounding area for receiving a signal generated by the nebulizing device 21. For example, the metal induction element 31 of the induction module 3 is partially exposed from the inner surrounding area of the medicine storage casing 10, so that the exposed induction portion 310 of the metal induction element 31 can be exposed in the medicine storage space 1000. Moreover, the second conductive structure 32 of the induction module 3 passes through the carrier seat 11, so that an electrode contact portion 320 of the second conductive structure 32 can be exposed outside the carrier seat 11 (that is to say, the electrode contact portion 320 of the second conductive structure 32 can be exposed from a bottom side of the carrier seat 11). In addition, when the metal induction element 31 is a horizontal metal induction element, the metal induction element 31 can be electrically connected to the second conductive structure 32 directly or through a conductive spring 33. However, the aforementioned details are disclosed for exemplary purposes only, and are not meant to limit the scope of the present disclosure.

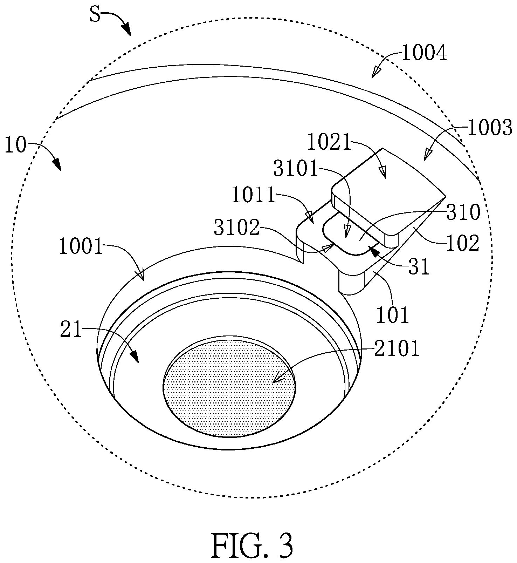

More particularly, for example, referring to FIG. 3, FIG. 4 and FIG. 5, the inner surrounding area has a first surrounding surface 1003 surrounding the first through hole 1001, and a second surrounding surface 1004 surrounding the first surrounding surface 1003. Moreover, the medicine storage casing 10 has a first protruding blocking portion 101 and a second protruding blocking portion 102 that are disposed on the first surrounding surface 1003, and the first protruding blocking portion 101 is connected between the first through hole 1001 and the second protruding blocking portion 102. In addition, an upper surface 3101 of the exposed induction portion 310 of the metal induction element 31 can be exposed by the first protruding blocking portion 101, and a side surface 3102 of the exposed induction portion 310 of the metal induction element 31 can be covered by the first protruding blocking portion 101. It should be noted that an upper surface 1011 of the first protruding blocking portion 101 can be flush with or lower than the upper surface 3101 of the exposed induction portion 310, and an upper surface 1021 of the second protruding blocking portion 102 can be located above the upper surface 3101 of the exposed induction portion 310. In addition, the upper surface 3101 of the exposed induction portion 310 of the at least one metal induction element 31 can be shown as circular, square, rectangular, arc or any shape. However, the aforementioned details are disclosed for exemplary purposes only, and are not meant to limit the scope of the present disclosure.

More particularly, referring to FIG. 1, FIG. 6 and FIG. 8, the first embodiment of the present disclosure further provides a portable nebulizer P which includes a nebulizer main body N and a vertical-type medicine containing and nebulizing assembly S.

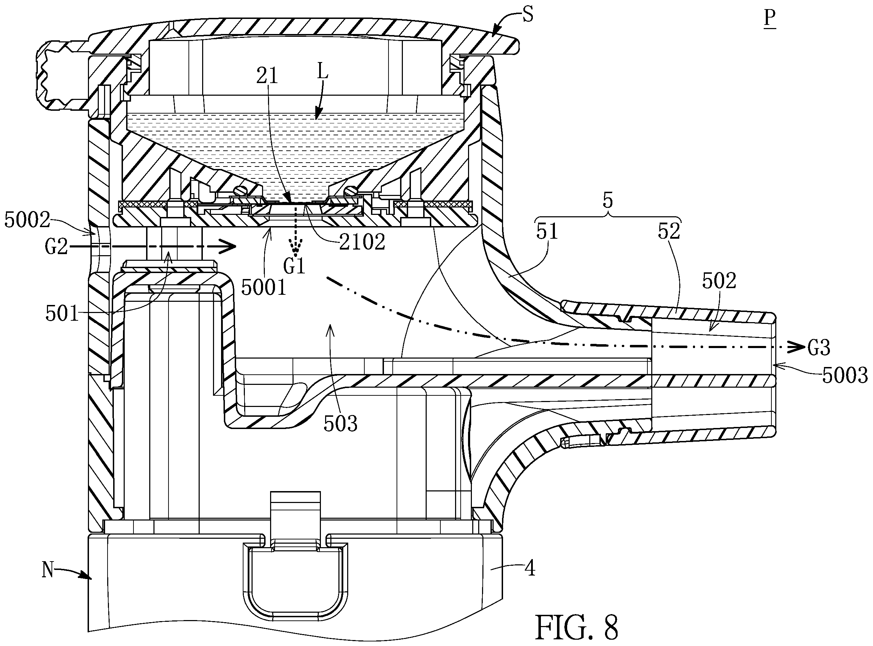

For example, referring to FIG. 1, FIG. 6 and FIG. 8, the nebulizer main body N includes a main body casing 4, a nebulizer subassembly 5 (such as a chamber providing subassembly) disposed on the main body casing 4, and a microprocessor 6 disposed in the main body casing 4. Therefore, when the vertical-type medicine containing and nebulizing assembly S is detachably or fixedly disposed on the nebulizer main body N, the nebulizer main body N can be configured to supply a power to the nebulizing device 21 through the first conductive structure 22, the nebulizing device 21 can be configured for nebulizing a predetermined medicinal liquid L (as shown in FIG. 8) contained in the medicine storage space 1000, and the induction module 3 can be electrically connected to the microprocessor 6 disposed inside the nebulizer main body N through the second conductive structure 32. However, the aforementioned details are disclosed for exemplary purposes only, and are not meant to limit the scope of the present disclosure.

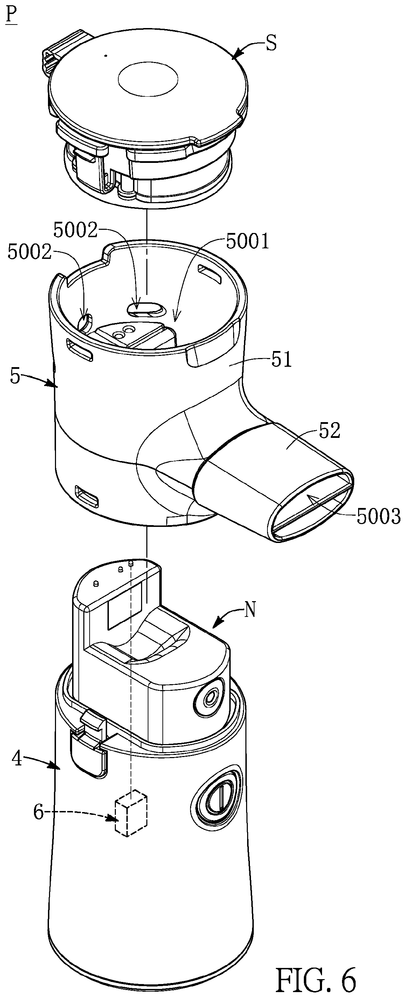

For example, referring to FIG. 6, FIG. 8 and FIG. 9, the nebulizer subassembly 5 includes a structural matching portion 51 disposed between the vertical-type medicine containing and nebulizing assembly S and the main body casing 4, and an extending nozzle portion 52 (such as a mouth piece portion) vertically and outwardly extending from the structural matching portion 51, and the nebulizer subassembly 5 has a nebulized aerosol entering opening 5001 disposed on the structural matching portion 51, a plurality of external air introducing openings 5002 disposed on the structural matching portion 51, and a nebulized aerosol spraying opening 5003 disposed on the extending nozzle portion 52. Moreover, each external air introducing opening 5002 and the nebulized aerosol spraying opening 5003 can be respectively located on a rear area (i.e., a rear end area) and a front area (i.e., a front end area) of the nebulizer subassembly 5, and the nebulized aerosol entering opening 5001 is located between the external air introducing opening 5002 and the nebulized aerosol spraying opening 5003. However, the aforementioned details are disclosed for exemplary purposes only, and are not meant to limit the scope of the present disclosure.

For example, referring to FIG. 6, FIG. 8 and FIG. 9, the nebulizer subassembly 5 has an external air guiding channel 501, a nebulized aerosol guiding channel 502 and an air mixing chamber 503 that are formed inside the nebulizer subassembly 5. The external air guiding channel 501 is communicated with the external air introducing openings 5002, the nebulized aerosol guiding channel 502 is communicated with the nebulized aerosol spraying opening 5003, and the air mixing chamber 503 is communicated with the external air guiding channel 501, the nebulized aerosol guiding channel 502, and an outlet opening 2102 of the nebulizing device 21. Therefore, after "a nebulized aerosol G1 (such as a nebulized fluid) outputted through the outlet opening 2102 of the nebulizing device 21" and "external air G2 (such as ambient air) introduced through the external air introducing openings 5002" can be mixed in the air mixing chamber 503 to form a mixed nebulized aerosol G3 (such as a mixed nebulized fluid), the mixed nebulized aerosol is guided through the nebulized aerosol guiding channel 502 and then sprayed out from the nebulized aerosol spraying opening 5003. It should be noted that according to different requirements, opening shapes of the external air introducing openings 5002 can be designed to be identical or different (such as at least two different shapes), opening areas of the external air introducing openings 5002 can be designed to be identical or different (such as at least two different areas), and center points of the external air introducing openings 5002 can be located at the same horizontal plane (or the same level) or different horizontal planes (such as at least two different levels). However, the aforementioned details are disclosed for exemplary purposes only, and are not meant to limit the scope of the present disclosure.

Therefore, when the volume of the predetermined medicinal liquid L in the medicine storage space 1000 is decreased to be unable to contact the exposed induction part 310 (or when the predetermined medicinal liquid L in the medicine storage space 1000 has been completely used up), the nebulizer main body N will stop supplying power to the nebulizing device 21 to achieve the effect of automatically turning off the nebulizing device 21 (or automatically turning off the portable nebulizer P). In a preferred embodiment, when the volume of the predetermined medicinal liquid L in the medicine storage space 1000 is decreased to be unable to contact the exposed induction part 310, the residual liquid volume of the predetermined medicinal liquid L will be equal to or less than 0.4 ml.

### [Second Embodiment]

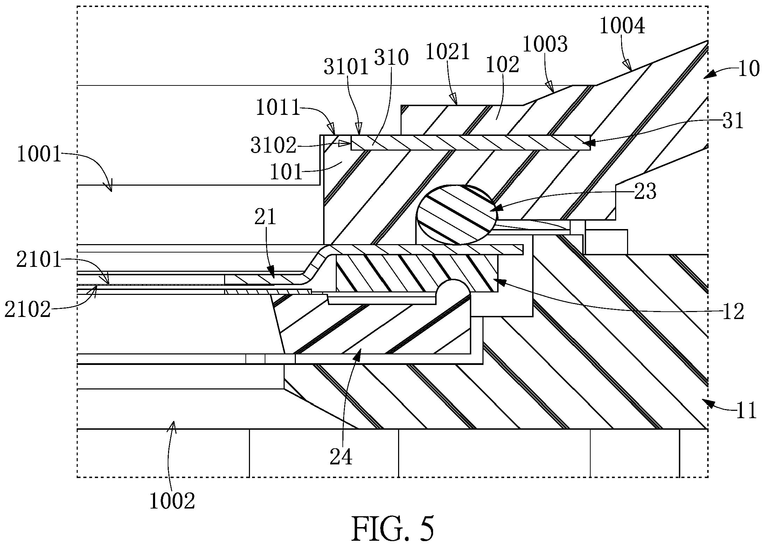

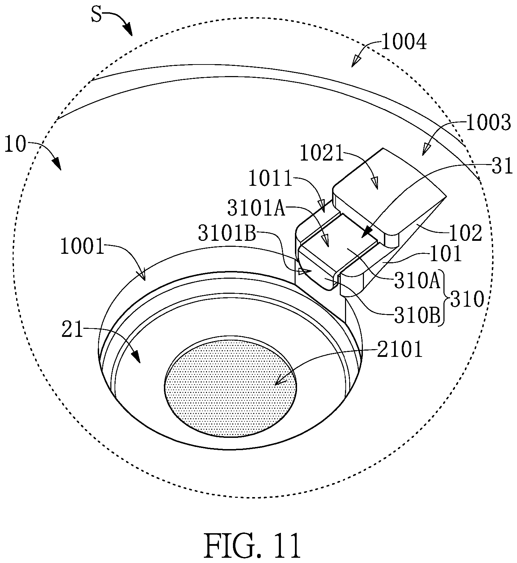

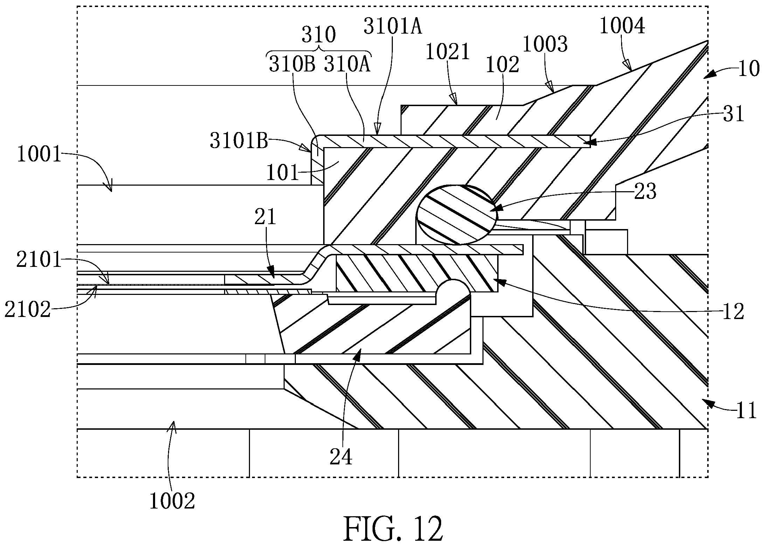

Referring to FIG. 11 and FIG. 12, a second embodiment of the present disclosure provides a vertical-type medicine containing and nebulizing assembly S. Comparing FIG. 11 with FIG. 3, and comparing FIG. 12 with FIG. 5, the main difference between the second embodiment and the first embodiment is as follows: in the second embodiment, the exposed induction portion 310 of the metal induction element 31 has an extending section 310A disposed on the first protruding blocking portion 101, and a bending section 310B extending downwardly from the extending section 310A (that is to say, the bending section 310B extends toward the first through hole 1001), and an upper surface 3101A of the extending section 310A and an upper surface 3101B of the bending section 310B are both exposed by the first protruding blocking portion 101.

For example, an upper surface 1011 of the first protruding blocking portion 101 can be flush with or lower than the upper surface 3101A of the extending section 310A of the exposed induction portion 310, and an upper surface 1021 of the second protruding blocking portion 102 can be located above upper surface 3101A of the extending section 310A of the exposed induction portion 310. However, the aforementioned details are disclosed for exemplary purposes only, and are not meant to limit the scope of the present disclosure.

### [Third Embodiment]

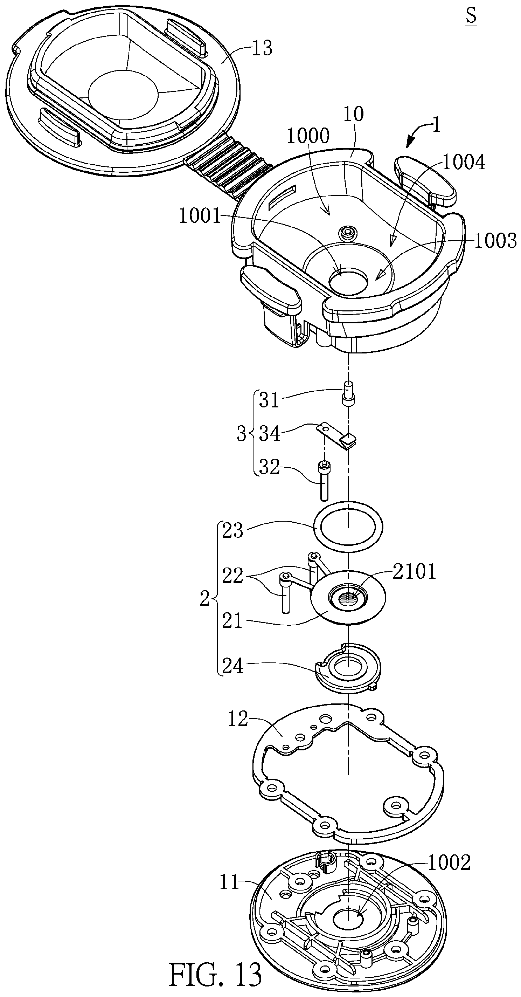

Referring to FIG. 11 to FIG. 17, a third embodiment of the present disclosure provides a vertical-type medicine containing and nebulizing assembly S. Comparing FIGS. 13 to 17 with FIGS. 1 to 5 respectively, the main difference between the third embodiment and the first embodiment is as follows: in the third embodiment, the medicine storage casing 10 has a protruding blocking portion 100 disposed on the second surrounding surface 1004, the protruding blocking portion 100 is away from the first through hole 1001 by a predetermined distance, and an upper surface 3101 and a top surrounding surface 3103 of the exposed induction portion 310 of the metal induction element 31 are exposed by the protruding blocking portion 100.

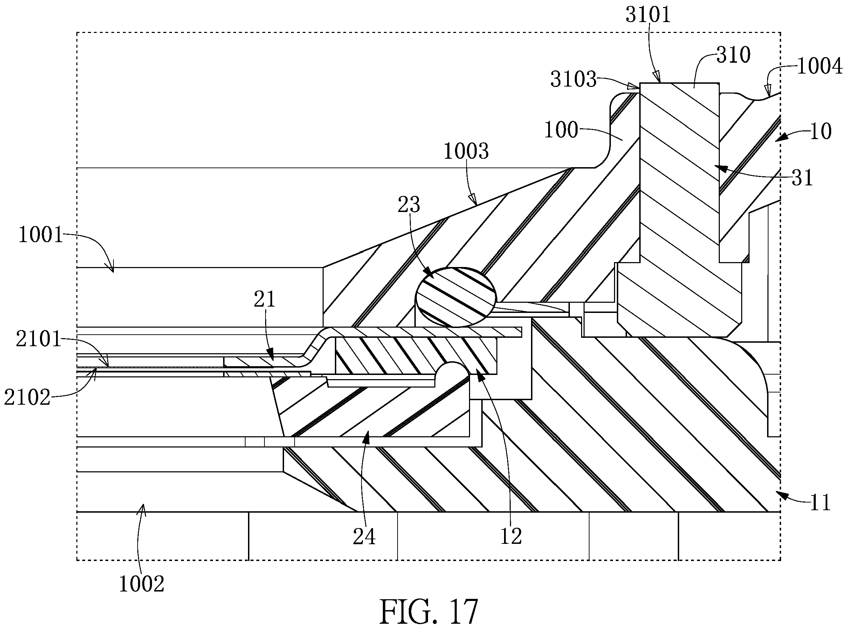

For example, referring to FIG. 13 and FIG. 17, when the metal induction element 31 is a vertical metal induction element, the metal induction element 31 can be electrically connected to the second conductive structure 32 through a conductive resilient sheet 34. In addition, the upper surface 3101 of the exposed induction portion 310 of the metal induction element 31 can be shown as circular or any shape. However, the aforementioned details are disclosed for exemplary purposes only, and are not meant to limit the scope of the present disclosure.

### [Fourth Embodiment]

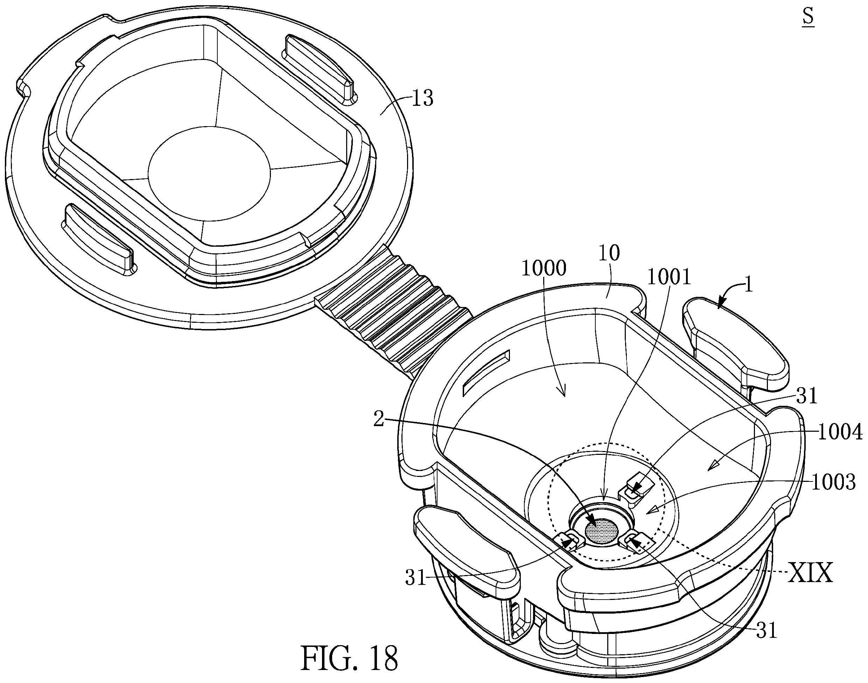

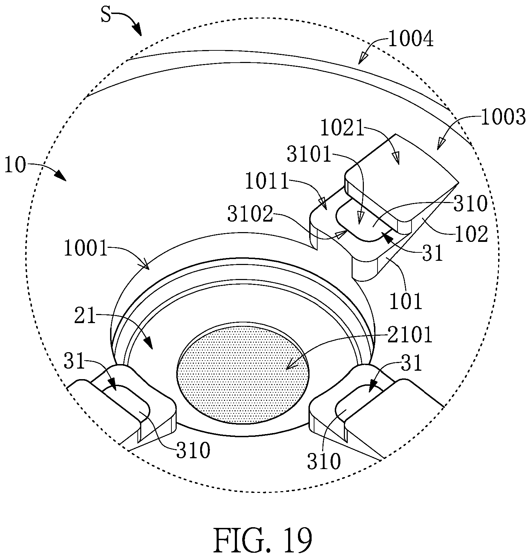

Referring to FIG. 18 and FIG. 17, a fourth embodiment of the present disclosure provides a vertical-type medicine containing and nebulizing assembly S. Comparing FIG. 18 with FIG. 2, and comparing FIG. 19 with FIG. 3, the main difference between the fourth embodiment and the first embodiment is as follows: in the fourth embodiment, when the number of the exposed induction portions 310 exposed on the inner surrounding area is at least three (for example, the at least three exposed induction portions 310 can be arranged at an equal angle or an unequal angle), the three exposed induction portions 310 can be located on the same horizontal plane and surround the first through hole 1001, and the exposed induction portions 310 can be disposed on the same metal induction element 31 or different metal induction elements 31. Thereby, when the nebulizing device 21 is configured to be used for the nebulization, if the nebulizer main body N is placed upright, the predetermined medicinal liquid L contained in the medicine storage space 1000 can simultaneously contact the exposed induction portions 310, so that the signal generated by the nebulizing device 21 can be inducted by the exposed induction portions 310. In addition, when the nebulizing device 21 is configured to be used for the nebulization, if the nebulizer main body N is placed in an inclined position (for example, the nebulizer main body N is inclined relative to a vertical line at an inclination angle greater than or equal to 30 degrees), the predetermined medicinal liquid L contained in the medicine storage space 1000 will only contact a part of the exposed induction portion 310, so that the signal generated by the nebulizing device 21 will not be inducted by the exposed induction portion 310 that does not contact the predetermined medicinal liquid L, and the missing signal will not be transmitted to the microprocessor 6 by the induction module 3. Therefore, when the microprocessor 6 fails to receive the corresponding signal, the nebulizer main body N will remind the user to confirm the use state.

### [Fifth Embodiment]

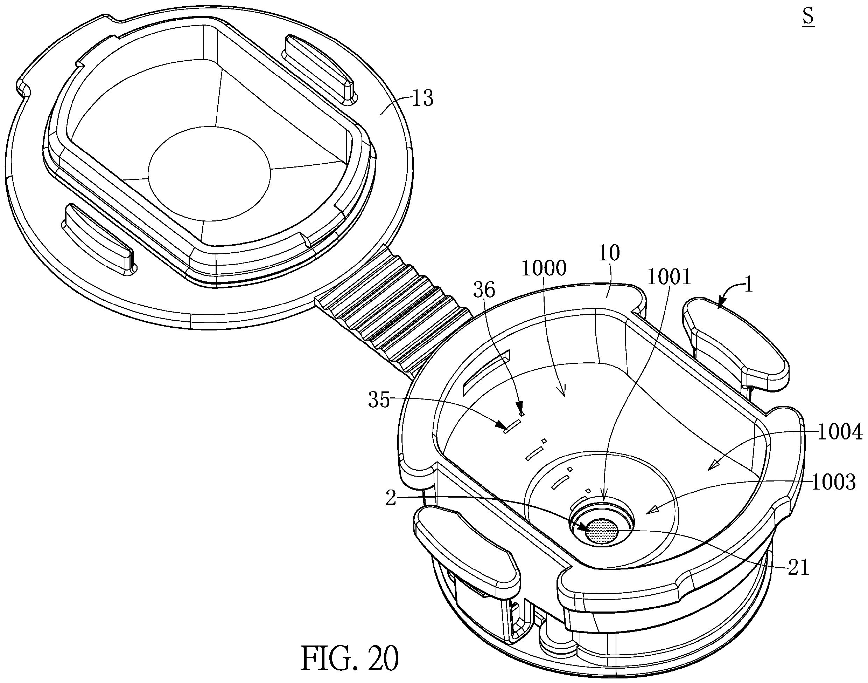

Referring to FIG. 20, a fifth embodiment of the present disclosure provides a vertical-type medicine containing and nebulizing assembly S. Comparing FIG. 20 with FIG. 2, the main difference between the fifth embodiment and the first embodiment is as follows: in the fifth embodiment, the inner surrounding area has a plurality of liquid level measurement points 35 (or liquid level measurement lines) and a plurality of liquid level induction portions 36, and the liquid level induction portions 36 are disposed adjacent to and along the liquid level measurement points 35. In addition, the liquid level measurement points 35 are not located on the same horizontal plane and can be arranged on the first surrounding surface 1003 and/or the second surrounding surface 1004, and the liquid level induction portions 36 are not located on the same horizontal plane and can be arranged on the first surrounding surface 1003 and/or the second surrounding surface 1004.

Therefore, except for the liquid level measurement points 35 that can be provided for the user to observe, the nebulizing device 21 can also optionally use the liquid level induction portions 36 to achieve the function of segmented quantitative medicinal liquid nebulization (each of the liquid level measurement points 35 can be a segmented quantitative nebulizing point of the medicinal liquid), so that the nebulizer main body N can stop supplying power to the nebulizing device 21 in order to achieve the effect of automatically turning off the nebulizing device 21 (or automatically turning off the portable nebulizer P) according to the segmental induction of the liquid level induction portions 36. It is worth noting that, for the liquid level measurement point 35 and the liquid level induction portion 36 that are adjacent to each other, the position of the liquid level induction portion 36 is slightly higher than the liquid level measurement point 35, so that the medicinal liquid can be inducted by the liquid level induction portion 36 before the medicinal liquid is lowered to the liquid level measurement point 35 or when the medicinal liquid is just located on the liquid level measurement point 35.

### [Beneficial Effects of the Embodiments]

One beneficial effect of the present disclosure is that in the portable nebulizer P and the vertical-type medicine containing and nebulizing assembly S thereof provided by the present disclosure, when the predetermined medicinal liquid contained in the medicine storage space 1000 is nebulized through the nebulizing device 21, the signal generated by the nebulizing device 21 can be transmitted to the microprocessor 6 through the induction module 3, by virtue of "the medicine storage module 1 including a medicine storage casing 10, and the medicine storage module 1 having a medicine storage space 1000 formed in the medicine storage casing 10 and a first through hole 1001 communicated with the medicine storage space 1000," "the nebulizing module 2 including a nebulizing device 21 disposed in the medicine storage module 1 and a first conductive structure 22 electrically connected to the nebulizing device 21," "the induction module 3 including a metal induction element 31 disposed in the medicine storage module 1 and a second conductive structure 32 electrically connected to the metal induction element 31," and "the medicine storage casing 10 having an inner surrounding area located in the medicine storage space 1000 and surrounding the first through hole 1001, and the metal induction element 31 having an exposed induction portion 310 exposed on the inner surrounding area for receiving the signal generated by the nebulizing device 21."

More particularly, when the volume of the predetermined medicinal liquid L in the medicine storage space 1000 is decreased to be unable to contact the exposed induction part 310 (or when the predetermined medicinal liquid L in the medicine storage space 1000 has been completely used up), the nebulizer main body N will stop supplying power to the nebulizing device 21 to achieve the effect of automatically turning off the nebulizing device 21 (or automatically turning off the portable nebulizer P).

The foregoing description of the exemplary embodiments of the disclosure has been presented only for the purposes of illustration and description and is not intended to be exhaustive or to limit the disclosure to the precise forms disclosed. Many modifications and variations are possible in light of the above teaching.

The embodiments were chosen and described in order to explain the principles of the disclosure and their practical application so as to enable others skilled in the art to utilize the disclosure and various embodiments and with various modifications as are suited to the particular use contemplated. Alternative embodiments will become apparent to those skilled in the art to which the present disclosure pertains without departing from its scope.

## Claims

1. A vertical-type medicine containing and nebulizing assembly (S), **characterized by** comprising:
a medicine storage module (1) including a medicine storage casing (10), wherein the medicine storage module (1) has a medicine storage space (1000) formed in the medicine storage casing (10) and a first through hole (1001) communicated with the medicine storage space (1000);
a nebulizing module (2) including a nebulizing device (21) disposed in the medicine storage module (1) and a first conductive structure (22) electrically connected to the nebulizing device (21); and
an induction module (3) including at least one metal induction element (31) disposed in the medicine storage module (1) and a second conductive structure (32) electrically connected to the at least one metal induction element (31);
wherein the medicine storage casing (10) has an inner surrounding area located in the medicine storage space (1000) and surrounding the first through hole (1001), and the at least one metal induction element (31) has at least one exposed induction portion (310) exposed on the inner surrounding area for receiving a signal generated by the nebulizing device (21);
**characterized in that** the number of the exposed induction portions (310) is multiple, and that the multiple exposed induction portions (310) surround the first through hole (1001) and are located on a same horizontal plane.

2. The vertical-type medicine containing and nebulizing assembly (S) according to claim 1, wherein:
the medicine storage module (1) includes a carrier seat (11) configured to cooperate with the medicine storage casing (10), a waterproof element (12) disposed between the medicine storage casing (10) and the carrier seat (11), and a movable cover (13) movably disposed on the medicine storage casing (10), the medicine storage module (1) has a second through hole (1002) passing through the carrier seat (11), and the medicine storage space (1000) is formed between the movable cover (13) and the medicine storage casing (10);
wherein the nebulizing module (2) includes a first waterproof washer (23) disposed between the medicine storage casing (10) and the nebulizing device (21), and a second waterproof washer (24) disposed between the carrier seat (11) and the nebulizing device (21), and the nebulizing device (21) has an inlet opening (2101) communicated with the first through hole (1001) and an outlet opening (2102) communicated with the second through hole (1002);
wherein the at least one metal induction element (31) of the induction module (3) is partially exposed from the inner surrounding area of the medicine storage casing (10), so that the exposed induction portion (310) of the at least one metal induction element (31) is exposed in the medicine storage space (1000), and the second conductive structure (32) of the induction module (3) passes through the carrier seat (11), so that an electrode contact portion (320) of the second conductive structure (32) is exposed outside the carrier seat (11);
wherein the at least one metal induction element (31) is a horizontal metal induction element or a vertical metal induction element; wherein, when the at least one metal induction element (31) is the horizontal metal induction element, the at least one metal induction element (31) is electrically connected to the second conductive structure (32) through a conductive spring (33); wherein, when the at least one metal induction element (31) is the vertical metal induction element, the at least one metal induction element (31) is electrically connected to the second conductive structure (32) through a conductive resilient sheet (34); and
wherein, when a predetermined medicinal liquid (L) contained in the medicine storage space (1000) is nebulized through the nebulizing device (21), the signal generated by the nebulizing device (21) is transmitted to a microprocessor (6) through the induction module (3).

3. The vertical-type medicine containing and nebulizing assembly (S) according to claim 1, wherein the inner surrounding area has a plurality of liquid level measurement points (35) and a plurality of liquid level induction portions (36) disposed adjacent to and along the liquid level measurement points (35), the liquid level measurement points (35) are not located on a same horizontal plane, and the liquid level induction portions (36) are not located on a same horizontal plane.

4. The vertical-type medicine containing and nebulizing assembly (S) according to claim 1, wherein:
the inner surrounding area has a surface surrounding the first through hole (1001), and the medicine storage casing (10) has a first protruding blocking portion (101) and a second protruding blocking portion (102) that are disposed on the surface of the inner surrounding area, and the first protruding blocking portion (101) is connected between the first through hole (1001) and the second protruding blocking portion (102); and
wherein an upper surface (3101) of the exposed induction portion (310) of the at least one metal induction element (31) is exposed by the first protruding blocking portion (101), and a side surface (3102) of the exposed induction portion (310) of the at least one metal induction element (31) is covered by the first protruding blocking portion (101).

5. The vertical-type medicine containing and nebulizing assembly (S) according to claim 1, wherein:
the inner surrounding area has a surface surrounding the first through hole (1001), and the medicine storage casing (10) has a first protruding blocking portion (101) and a second protruding blocking portion (102) that are disposed on the surface of the inner surrounding area, and the first protruding blocking portion (101) is connected between the first through hole (1001) and the second protruding blocking portion (102); and
wherein the exposed induction portion (310) of the at least one metal induction element (31) has an extending section (310A) disposed on the first protruding blocking portion (101), and a bending section (310B) extending downwardly from the extending section (310A), and an upper surface (3101A) of the extending section (310A) and an upper surface (3101B) of the bending section (310B) are both exposed by the first protruding blocking portion (101).

6. The vertical-type medicine containing and nebulizing assembly (S) according to claim 1, wherein:
the inner surrounding area has a surface surrounding the first through hole (1001), and the medicine storage casing (10) has a protruding blocking portion (100) disposed on the surface of the inner surrounding area; and
wherein, an upper surface (3101) and a top surrounding surface (3103) of the exposed induction portion (310) of the at least one metal induction element (31) are exposed by the protruding blocking portion (100).

## Patentansprüche

1. Vertikaler-Typ-Arzneimittel-Unterbringung-und-Vernebelung-Vorrichtung (S), **dadurch gekennzeichnet, dass** sie aufweist:
ein Arzneimittelaufbewahrungsmodul (1), welches ein Arzneimittelaufbewahrungsgehäuse (10) aufweist, wobei das Arzneimittelaufbewahrungsmodul (1) einen Arzneimittelaufbewahrungsraum (1000), welcher in dem Arzneimittelaufbewahrungsgehäuse (10) gebildet ist, und ein erstes Durchgangsloch (1001) hat, welches mit dem Arzneimittelaufbewahrungsraum (1000) in Verbindung steht;
ein Vernebelungsmodul (2), welches eine Vernebelungsvorrichtung (21), welche in dem Arzneimittelaufbewahrungsmodul (1) angeordnet ist, und eine erste leitfähige Struktur (22) aufweist, welche elektrisch mit der Vernebelungsvorrichtung (21) verbunden ist; und
ein Induktionsmodul (3), welches mindestens ein metallisches Induktionselement (31), welches in dem Arzneimittelaufbewahrungsmodul (1) angeordnet ist, und eine zweite leitfähige Struktur (32) aufweist, welche elektrisch mit dem mindestens einen metallischen Induktionselement (31) verbunden ist;
wobei:
das Arzneimittelaufbewahrungsgehäuse (10) einen inneren Umgebungsbereich hat, welcher sich in dem Arzneimittelaufbewahrungsraum (1000) befindet und das erste Durchgangsloch (1001) umgibt, und wobei das mindestens eine metallische Induktionselement (31) mindestens einen freiliegenden Induktionsabschnitt (310) hat, welcher an dem inneren Umgebungsbereich freiliegend ist zum Empfangen eines Signals, welches mittels der Vernebelungsvorrichtung (21) erzeugt wird;
**dadurch gekennzeichnet, dass** die Anzahl der freiliegenden Induktionsabschnitte (310) eine Mehrzahl ist, und dass die mehreren freiliegenden Induktionsabschnitte (310) das erste Durchgangsloch (1001) umgeben und an einer gleichen horizontalen Ebene angeordnet sind.

2. Vertikaler-Typ-Arzneimittel-Unterbringung-und-Vernebelung-Vorrichtung (S) gemäß Anspruch 1, wobei:
das Arzneimittelaufbewahrungsmodul (1) einen Trägersitz (11), welcher so konfiguriert ist, dass er mit dem Arzneimittelaufbewahrungsgehäuse (10) zusammenwirkt, ein wasserdichtes Element (12), welches zwischen dem Arzneimittelaufbewahrungsgehäuse (10) und dem Trägersitz (11) angeordnet ist, und eine bewegbare Abdeckungsvorrichtung (13) aufweist, welche bewegbar an dem Arzneimittelaufbewahrungsgehäuse (10) angeordnet ist, wobei das Arzneimittelaufbewahrungsmodul (1) ein zweites Durchgangsloch (1002) hat, welches durch den Trägersitz (11) hindurch verläuft, und wobei der Arzneimittelaufbewahrungsraum (1000) zwischen der bewegbaren Abdeckungsvorrichtung (13) und dem Arzneimittelaufbewahrungsgehäuse (10) gebildet ist;
wobei das Vernebelungsmodul (2) eine erste wasserdichte Unterlegscheibe (23), welche zwischen dem Arzneimittelaufbewahrungsgehäuse (10) und der Vernebelungsvorrichtung (21) angeordnet ist, und eine zweite wasserdichte Unterlegscheibe (24) aufweist, welche zwischen dem Trägersitz (11) und der Vernebelungsvorrichtung (21) angeordnet ist, und wobei die Vernebelungsvorrichtung (21) eine Einlassöffnung (2101), welche mit dem ersten Durchgangsloch (1001) in Verbindung steht, und eine Auslassöffnung (2102) hat, welche mit dem zweiten Durchgangsloch (1002) in Verbindung steht;
wobei das mindestens eine metallische Induktionselement (31) des Induktionsmoduls (3) teilweise von dem inneren Umgebungsbereich des Arzneimittelaufbewahrungsgehäuses (10) aus freiliegend ist, so dass der freiliegende Induktionsabschnitt (310) des mindestens einen metallischen Induktionselements (31) in dem Arzneimittelaufbewahrungsraum (1000) freiliegend ist, und wobei die zweite leitfähige Struktur (32) des Induktionsmoduls (3) durch den Trägersitz (11) hindurch verläuft, so dass ein Elektrodenkontaktabschnitt (320) der zweiten leitfähigen Struktur (32) außerhalb von dem Trägersitz (11) freiliegend ist;
wobei das mindestens eine metallische Induktionselement (31) ein horizontales metallisches Induktionselement oder ein vertikales metallisches Induktionselement ist; wobei, wenn das mindestens eine metallische Induktionselement (31) das horizontale metallische Induktionselement ist, das mindestens eine metallische Induktionselement (31) mit der zweiten leitfähigen Struktur (32) durch eine leitfähige Feder (33) hindurch elektrisch verbunden ist; wobei, wenn das mindestens eine metallische Induktionselement (31) das vertikale metallische Induktionselement ist, das mindestens eine metallische Induktionselement (31) mit der zweiten leitfähigen Struktur (32) durch ein leitfähiges elastisches Plättchen (34) hindurch elektrisch verbunden ist; und
wobei, wenn eine vorbestimmte medizinische Flüssigkeit (L), welche in dem Arzneimittelaufbewahrungsraum (1000) untergebracht ist, durch die Vernebelungsvorrichtung (21) vernebelt wird, das Signal, welches mittels der Vernebelungsvorrichtung (21) erzeugt wird, durch das Induktionsmodul (3) an einen Mikroprozessor (6) übertragen wird.

3. Vertikaler-Typ-Arzneimittel-Unterbringung-und-Vernebelung-Vorrichtung (S) gemäß Anspruch **1,** wobei der innere Umgebungsbereich mehrere Flüssigkeitspegelmesspunkte (35) und mehrere Flüssigkeitspegelinduktionsabschnitte (36) hat, welche benachbart zu und entlang von den Flüssigkeitspegelmesspunkten (35) angeordnet sind, wobei sich die Flüssigkeitspegelmesspunkte (35) nicht an einer gleichen horizontalen Ebene befinden, und wobei sich die Flüssigkeitspegelinduktionsabschnitte (36) nicht an einer gleichen horizontalen Ebene befinden.

4. Vertikaler-Typ-Arzneimittel-Unterbringung-und-Vernebelung-Vorrichtung (S) gemäß Anspruch 1, wobei:
der innere Umgebungsbereich eine Fläche hat, welche das erste Durchgangsloch (1001) umgibt, und wobei das Arzneimittelaufbewahrungsgehäuse (10) einen ersten hervorstehenden Blockierabschnitt (101) und einen zweiten hervorstehenden Blockierabschnitt (102) hat, welche an der Fläche des inneren Umgebungsbereichs angeordnet sind, und wobei der erste hervorstehende Blockierabschnitt (101) zwischen dem ersten Durchgangsloch (1001) und dem zweiten hervorstehenden Blockierabschnitt (102) verbunden ist; und
wobei eine obere Fläche (3101) des freiliegenden Induktionsabschnitts (310) des mindestens einen metallischen Induktionselements (31) mittels des ersten hervorstehenden Blockierabschnitts (101) freiliegend ist, und wobei eine Seitenfläche (3102) des freiliegenden Induktionsabschnitts (310) des mindestens einen metallischen Induktionselements (31) mittels des ersten hervorstehenden Blockierabschnitts (101) bedeckt ist.

5. Vertikaler-Typ-Arzneimittel-Unterbringung-und-Vernebelung-Vorrichtung (S) gemäß Anspruch 1, wobei:
der innere Umgebungsbereich eine Fläche hat, welche das erste Durchgangsloch (1001) umgibt, und wobei das Arzneimittelaufbewahrungsgehäuse (10) einen ersten hervorstehenden Blockierabschnitt (101) und einen zweiten hervorstehenden Blockierabschnitt (102) hat, welche an der Fläche des inneren Umgebungsbereichs angeordnet sind, und wobei der erste hervorstehende Blockierabschnitt (101) zwischen dem ersten Durchgangsloch (1001) und dem zweiten hervorstehenden Blockierabschnitt (102) verbunden ist; und
wobei der freiliegende Induktionsabschnitt (310) des mindestens einen metallischen Induktionselements (31) einen Erstreckungsabschnitt (310A), welcher an dem ersten hervorstehenden Blockierabschnitt (101) angeordnet ist, und einen Biegeabschnitt (310B) hat, welcher sich von dem Erstreckungsabschnitt (310A) aus nach unten hin erstreckt, und wobei sowohl eine obere Fläche (3101A) des Erstreckungsabschnitts (310A) als auch eine obere Fläche (3101B) des Biegebeabschnitts (310B) mittels des ersten hervorstehenden Blockierabschnitts (101) freiliegend sind.

6. Vertikaler-Typ-Arzneimittel-Unterbringung-und-Vernebelung-Vorrichtung (S) gemäß Anspruch 1, wobei:
der innere Umgebungsbereich eine Fläche hat, welche das erste Durchgangsloch (1001) umgibt, und wobei das Arzneimittelaufbewahrungsgehäuse (10) einen hervorstehenden Blockierabschnitt (100) hat, welcher an der Fläche des inneren Umgebungsbereichs angeordnet ist; und
wobei eine obere Fläche (3101) und eine Oben-Umgebungsfläche (3103) des freiliegenden Induktionsabschnitts (310) des mindestens einen metallischen Induktionselements (31) mittels des hervorstehenden Blockierabschnitts (100) freiliegend sind.

## Revendications

1. Ensemble (S) de type vertical pour contenir et nébuliser un médicament, **caractérisé en ce qu'**il comprend :
un module de stockage de médicament (1) comprenant un boîtier de stockage de médicament (10), le module de stockage de médicament (1) ayant un espace de stockage de médicament (1000) formé dans le boîtier de stockage de médicament (10) et un premier trou de passage (1001) mis en communication avec l'espace de stockage de médicament (1000) ;
un module de nébulisation (2) comprenant un dispositif de nébulisation (21) disposé dans le module de stockage de médicament (1) et une première structure conductrice (22) connectée électriquement au dispositif de nébulisation (21) ; et
un module d'induction (3) comprenant au moins un élément d'induction métallique (31) disposé dans le module de stockage de médicament (1) et une seconde structure conductrice (32) connectée électriquement à l'au moins un élément d'induction métallique (31) ;
le boîtier de stockage de médicament (10) ayant une zone environnante intérieure située dans l'espace de stockage de médicament (1000) et entourant le premier trou de passage (1001), et l'au moins un élément d'induction métallique (31) ayant au moins une partie d'induction exposée (310) exposée sur la zone environnante intérieure pour recevoir un signal généré par le dispositif de nébulisation (21) ;
**caractérisé en ce que** le nombre de parties d'induction exposées (310) est multiple, et **en ce que** les multiples parties d'induction exposées (310) entourent le premier trou de passage (1001) et sont situées sur un même plan horizontal.

2. Ensemble (S) de type vertical pour contenir et nébuliser un médicament selon la revendication 1,
le module de stockage de médicament (1) comprenant un siège de support (11) configuré pour coopérer avec le boîtier de stockage de médicament (10), un élément étanche (12) disposé entre le boîtier de stockage de médicament (10) et le siège de support (11), et un couvercle mobile (13) disposé de manière mobile sur le boîtier de stockage de médicament (10), le module de stockage de médicament (1) ayant un deuxième trou de passage (1002) traversant le siège de support (11), et l'espace de stockage de médicament (1000) étant formé entre le couvercle mobile (13) et le boîtier de stockage de médicament (10) ;
le module de nébulisation (2) comprenant une première rondelle étanche (23) disposée entre le boîtier de stockage de médicament (10) et le dispositif de nébulisation (21), et une seconde rondelle étanche (24) disposée entre le siège porteur (11) et le dispositif de nébulisation (21), et le dispositif de nébulisation (21) ayant une ouverture d'entrée (2101) mise en communication avec le premier trou de passage (1001) et une ouverture de sortie (2102) mise en communication avec le second trou de passage (1002) ;
l'au moins un élément d'induction métallique (31) du module d'induction (3) étant partiellement exposé à partir de la zone environnante intérieure du boîtier de stockage de médicament (10), de sorte que la partie d'induction exposée (310) de l'au moins un élément d'induction métallique (31) est exposée dans l'espace de stockage de médicament (1000), et la seconde structure conductrice (32) du module d'induction (3) traverse le siège de support (11), de sorte qu'une partie de contact d'électrode (320) de la seconde structure conductrice (32) est exposée à l'extérieur du siège de support (11) ;
l'au moins un élément d'induction métallique (31) étant un élément d'induction métallique horizontal ou un élément d'induction métallique vertical ;, lorsque l'au moins un élément d'induction métallique (31) est l'élément d'induction métallique horizontal, l'au moins un élément d'induction métallique (31) étant connecté électriquement à la seconde structure conductrice (32) par l'intermédiaire d'un ressort conducteur (33) ; lorsque l'au moins un élément d'induction métallique (31) est l'élément d'induction métallique vertical, l'au moins un élément d'induction métallique (31) étant connecté électriquement à la seconde structure conductrice (32) par l'intermédiaire d'une feuille résiliente conductrice (34) ; et
lorsqu'un liquide médicinal prédéterminé (L) contenu dans l'espace de stockage de médicament (1000) est nébulisé par le dispositif de nébulisation (21), le signal généré par le dispositif de nébulisation (21) étant transmis à un microprocesseur (6) par l'intermédiaire du module d'induction (3).

3. Ensemble (S) de type vertical pour contenir et nébuliser un médicament selon la revendication 1, la zone environnante intérieure ayant une pluralité de points de mesure du niveau de liquide (35) et une pluralité de parties d'induction du niveau de liquide (36) disposées à côté et le long des points de mesure du niveau de liquide (35), les points de mesure du niveau de liquide (35) n'étant pas situés sur un même plan horizontal, et les parties d'induction du niveau de liquide (36) n'étant pas situées sur un même plan horizontal.

4. Ensemble (S) de type vertical pour contenir et nébuliser un médicament selon la revendication 1,
la zone environnante intérieure ayant une surface entourant le premier trou de passage (1001), et le boîtier de stockage de médicament (10) ayant une première partie de blocage saillante (101) et une deuxième partie de blocage saillante (102) qui sont disposées sur la surface de la zone environnante intérieure, et la première partie de blocage saillante (101) étant connectée entre le premier trou de passage (1001) et la deuxième partie de blocage saillante (102) ; et
une surface supérieure (3101) de la partie d'induction exposée (310) de l'au moins un élément d'induction métallique (31) étant exposée par la première partie de blocage saillante (101), et une surface latérale (3102) de la partie d'induction exposée (310) de l'au moins un élément d'induction métallique (31) étant couverte par la première partie de blocage saillante (101).

5. Ensemble (S) de type vertical pour contenir et nébuliser un médicament selon la revendication 1,
la zone environnante intérieure ayant une surface entourant le premier trou de passage (1001), et le boîtier de stockage de médicament (10) ayant une première partie de blocage saillante (101) et une deuxième partie de blocage saillante (102) qui sont disposées sur la surface de la zone environnante intérieure, et la première partie de blocage saillante (101) étant connectée entre le premier trou de passage (1001) et la deuxième partie de blocage saillante (102) ; et
la partie d'induction exposée (310) de l'au moins un élément d'induction métallique (31) ayant une section d'extension (310A) disposée sur la première partie de blocage saillante (101), et une section de flexion (310B) s'étendant vers le bas à partir de la section d'extension (310A), et une surface supérieure (3101A) de la section d'extension (310A) et une surface supérieure (3101B) de la section de flexion (310B) étant toutes les deux exposées par la première partie de blocage saillante (101).

6. Ensemble (S) de type vertical pour contenir et nébuliser un médicament selon la revendication 1,
la zone environnante intérieure ayant une surface entourant le premier trou de passage (1001), et le boîtier de stockage de médicament (10) ayant une partie de blocage saillante (100) disposée sur la surface de la zone environnante intérieure ; et
une surface supérieure (3101) et une surface environnante supérieure (3103) de la partie d'induction exposée (310) de l'au moins un élément d'induction métallique (31) étant exposées par la partie de blocage saillante (100).
